(19) **Europäisches Patentamt / European Patent Office / Office européen des brevets**

(11) **EP 4 640 200 A1**

(12) **EUROPEAN PATENT APPLICATION**
published in accordance with Art. 153(4) EPC

(43) Date of publication:
29.10.2025 Bulletin 2025/44

(21) Application number: 23907214.3

(22) Date of filing: 22.12.2023

(51) International Patent Classification (IPC):
$A61K\ 6/833^{(2020.01)}$  $A61C\ 13/003^{(2006.01)}$
$A61K\ 6/818^{(2020.01)}$  $A61K\ 6/822^{(2020.01)}$
$A61L\ 27/10^{(2006.01)}$  $C03C\ 3/062^{(2006.01)}$
$C03C\ 3/078^{(2006.01)}$  $C03C\ 3/085^{(2006.01)}$
$C03C\ 3/087^{(2006.01)}$  $C04B\ 35/18^{(2006.01)}$

(52) Cooperative Patent Classification (CPC):
A61C 13/26; A61K 6/818; A61K 6/822;
A61K 6/833; A61L 27/10; C03C 3/062;
C03C 3/078; C03C 3/085; C03C 3/087; C04B 35/18

(86) International application number:
PCT/JP2023/046285

(87) International publication number:
WO 2024/135854 (27.06.2024 Gazette 2024/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.12.2022 JP 2022205842

(71) Applicant: Kuraray Noritake Dental Inc.
Kurashiki-shi, Okayama 710-0801 (JP)

(72) Inventors:
• SHIBATA, Keizo
Miyoshi-shi, Aichi 470-0293 (JP)
• KATO, Makiko
Miyoshi-shi, Aichi 470-0293 (JP)
• EMOTO, Tomohiro
Miyoshi-shi, Aichi 470-0293 (JP)

(74) Representative: D Young & Co LLP
3 Noble Street
London EC2V 7BQ (GB)

(54) **DENTAL PORCELAIN**

(57) The present invention relates to a dental porcelain containing $SiO_2$, $CeO_2$, $ZrO_2$, and an oxide of an alkaline earth metal, having a content of the $SiO_2$ of 30.0 to 50.0% by mass based on 100% by mass of the dental porcelain, and a content of the $CeO_2$ of 10.0 to 50.0% by mass based on 100% by mass of the dental porcelain; and a method for producing a dental prosthesis, including a step of coating the dental porcelain on a zirconia base material, and a step of firing the dental porcelain at 880 to 980°C.

EP 4 640 200 A1

**Description**

Technical Field

The present invention relates to a dental porcelain.

Background Art

Dental opaque porcelains are used mainly for the purposes of shielding the metallic color of frames or the abutment tooth color visible through frames.

For example, a metal-fusing porcelain necessarily shields the base metallic color, and therefore opaque porcelains have been conventionally available in the product lineup, which enable the production of a highly aesthetic prosthesis with no visible metallic color on the dental crown surface.

In recent years, for example, ceramics, such as a zirconia material, have transparency and color tone that are similar to the natural teeth, and are becoming an essential material for producing a dental crown that necessarily has aesthetics.

On the other hand, the spread of a ceramic product, particularly a zirconia material, having high strength and high transparency in recent years brings about an issue that the base metallic color cannot be sufficiently shielded thereby, and an opaque porcelain having a high shielding capability is being demanded for the ceramics-fusing porcelain, such as the zirconia material.

Furthermore, while the ordinary colored porcelain powder tends to achieve a shielding capability as compared to a white porcelain, a user liking bleached color tends to dislike a colored prosthesis. The bleached color with high whiteness degree demanded for the ceramic prosthesis formed of zirconia or the like often fail to provide a strong shielding capability, and for example, in the case using an implant or an abutment, the base metallic color tends to appear on the dental crown surface, and therefore a prosthesis having higher aesthetics is being demanded.

For example, PTL 1 describes a dental opaque porcelain containing 15 to 50% by weight of $CeO_2$ and 40 to 50% by weight of aluminosilicate glass, as a dental metal shielding material for shielding the metallic color of a dental metal base material in fusing.

PTL 2 describes a porcelain composition for producing an all ceramic dental crown restoration material, which is a porcelain composition set constituted by two layers mounted and fired on an all ceramic core, in which the first layer mounted and fired on the all ceramic core is a first layer composition of a porcelain for a ceramic dental crown obtained by blending two or more kinds of glass frits formed of a glass composition containing 40.0 to 60.0% by mass of $SiO_2$, 8.0 to 14.0% by mass of $Al_2O_3$, 0.5 to 4.0% by mass of $B_2O_3$, 3.0 to 6.0% by mass of $Na_2O$, 5.0 to 9.0% by mass of $K_2O$, 0.5 to 3.0% by mass of CaO, 0.1 to 2.0% by mass of MgO, and 5.0 to 15.0% by mass of $ZrO_2$, and the second layer mounted and fired on the first layer is constituted by a second layer composition of a porcelain for a ceramic dental crown obtained by blending two or more kinds of glass frits formed of a glass composition containing 50.0 to 70.0% by mass of $SiO_2$, 12.0 to 21.0% by mass of $Al_2O_3$, 2.0 to 9.0% by mass of $B_2O_3$, 0.05 to 0.2% by mass of $Li_2O$, 4.0 to 12.0% by mass of $Na_2O$, 4.0 to 12.0% by mass of $K_2O$, 0.5 to 5.0% by mass of CaO, and 0.1 to 2.0% by mass of MgO.

Citation List

Patent Literatures

PTL 1: JP 10-94550 A

PTL 2: JP 2007-126360 A

Summary of Invention

Technical Problem

However, the dental opaque porcelain described in PTL 1 is a porcelain that is used by fusing on a dental noble metal material (such as Au and an Au-Pd alloy), and in the case where the porcelain is fused on a ceramic base material, there are problems including cracks and breakages occurring in the porcelain.

The porcelain composition described in PTL 2 is a composition of a porcelain for a ceramic dental crown, and the porcelain can be fused on a ceramic base material, but it has been found that the shielding capability thereof is insufficient.

A porcelain may be colored black, yellow, or the like depending on the composition of the porcelain, and for producing a prosthesis having good whiteness degree and higher aesthetics, it is necessary to suppress the coloration of the porcelain, in addition of the excellent shielding capability.

Under the circumstances, an object of the present invention is to provide a dental porcelain that can suppress the occurrence of cracks in fusing on a ceramic base material, has an excellent shielding capability after firing, and can suppress the coloration after firing.

Solution to Problem

As a result of the earnest investigations by the present inventors, it has been found that the use of a dental porcelain having a specific composition can solve the problem, and thus the present invention has been completed.

Specifically, the present invention encompasses the following inventions.

[1] A dental porcelain containing $SiO_2$, $CeO_2$, $ZrO_2$, and an oxide of an alkaline earth metal, having

a content of the $SiO_2$ of 30.0 to 50.0% by mass based on 100% by mass of the dental porcelain, and
a content of the $CeO_2$ of 10.0 to 50.0% by mass based on 100% by mass of the dental porcelain.

[2] The dental porcelain according to the item [1], in which the dental porcelain has a content of the $ZrO_2$ of 5.0 to 16.0% by mass based on 100% by mass of the dental porcelain.

[3] The dental porcelain according to the item [1] or [2], which further contains $Al_2O_3$, $K_2O$, and $Na_2O$, and has

a content of the $Al_2O_3$ of 2.0 to 8.0% by mass based on 100% by mass of the dental porcelain,
a content of the $K_2O$ of 1.0 to 7.0% by mass based on 100% by mass of the dental porcelain, and
a content of the $Na_2O$ of 4.0 to 10.0% by mass based on 100% by mass of the dental porcelain.

[4] The dental porcelain according to any one of the items [1] to [3], in which the dental porcelain contains at least CaO and BaO as the oxide of an alkaline earth metal, and has

a content of the CaO of 0.2 to 2.0% by mass based on 100% by mass of the dental porcelain, and
a content of the BaO of 1.0 to 8.0% by mass based on 100% by mass of the dental porcelain.

[5] The dental porcelain according to any one of the items [1] to [4], in which the dental porcelain has a total content of the $SiO_2$, the $CeO_2$, the $ZrO_2$, and the oxide of an alkaline earth metal of 70.0 to 95.0% by mass based on 100% by mass of the dental porcelain.

[6] The dental porcelain according to any one of the items [1] to [5], in which the $CeO_2$ is $CeO_2$ that is derived from an opaquer.

[7] The dental porcelain according to any one of the items [1] to [6], in which a fired material obtained by firing the dental porcelain at a maximum firing temperature of 930°C has a thermal expansion coefficient at 30 to 450°C of $7.8 \times 10^{-6}$/°C or more and less than $10.0 \times 10^{-6}$ /°C measured according to ISO 6872 (2015) at a temperature rise rate of 10°C/min.

[8] The dental porcelain according to any one of the items [1] to [7], in which a measurement specimen having a thickness of 0.30 mm of a fired material obtained by firing the dental porcelain at a maximum firing temperature of 930°C has a transmittance of light having a wavelength of 555 nm of 10.0% or less in a thickness direction.

[9] The dental porcelain according to any one of the items [1] to [8], in which a test piece having a thickness of 1.20 mm of a fired material obtained by firing the dental porcelain at a maximum firing temperature of 930°C has a brightness L* of 90.0 or more obtained through colorimetry in the L*a*b* color space.

[10] A method for producing a dental prosthesis, including a step of coating the dental porcelain according to any one of the items [1] to [9] on a zirconia base material, and a step of firing the dental porcelain at 880 to 980°C.

Advantageous Effect of Invention

The present invention can provide a dental porcelain that can suppress the occurrence of cracks in fusing on a ceramic base material, has an excellent shielding capability after firing, and can suppress the coloration after firing.

Description of Embodiments

The present invention will be described in detail with reference to embodiments below.

In the description herein, the upper limit values and the lower limit values of the numerical ranges (for example, the contents of the components, the values and the properties calculated from the components, and the production conditions) can be optionally combined.

Specifically, in the description herein, the lower limit values and the upper limit values described in a stepwise manner each can be independently combined. For example, from the description "preferably 10 to 90, and more preferably 30 to 60" for the same item, the "preferred lower limit value (10)" and the "more preferred upper limit value (60)" can be combined to provide a range of "10 to 60".

As for the numerical ranges, for example, based on the description "preferably 10 to 90, and more preferably 30 to 60", only the lower limit value can be defined as "10 or more" or "30 or more" with no particular definition of the upper limit value, and similarly only the upper limit value can be defined as "90 or less" or "60 or less" with no particular definition of the lower limit value.

Unless otherwise indicated, the simple description "XX to YY" as a numerical range means a range of XX or more and YY or less. For example, the description "10 to 90" means a range of 10 or more and 90 or less.

As similar to as described above, for example, from the descriptions of "preferably 10 or more, and more preferably 30 or more" and "preferably 90 or less, and more preferably 60 or less" for the same item, the "preferred lower limit value (10)" and the "more preferred upper limit value (60)" can be combined to provide a range of" 10 to 60". As similar to as described above, only the lower limit value can be defined as "10 or more" or "30 or more", and similarly only the upper limit value can be defined as "90 or less" or "60 or less". The same is applied to the case where the upper end of the numerical range is "less than".

In the description herein, the term "porcelain" means a "dental porcelain" unless otherwise indicated.

[Dental Porcelain]

The dental porcelain as one embodiment of the present invention contains $SiO_2$, $CeO_2$, $ZrO_2$, and an oxide of an alkaline earth metal, and has a content of the $SiO_2$ of 30.0 to 50.0% by mass based on 100% by mass of the dental porcelain, and a content of the $CeO_2$ of 10.0 to 50.0% by mass based on 100% by mass of the dental porcelain.

The dental porcelain exhibits the effects of the present invention described above by satisfying these requirements.

<Content of $CeO_2$>

The content of $CeO_2$ that is 10.0% by mass or more based on 100% by mass of the dental porcelain can enhance the shielding capability of the dental porcelain. The content of $CeO_2$ that is 50.0% by mass or less based on 100% by mass of the dental porcelain can prevent the thermal expansion coefficient of the dental porcelain from becoming too high, and can suppress the occurrence of cracks in fusing on a ceramic base material.

From the standpoint, the content of $CeO_2$ is preferably 15.0 to 45.0% by mass, more preferably 18.0 to 40.0% by mass, and further preferably 19.0 to 31.0% by mass, based on 100% by mass of the dental porcelain.

<Content of $SiO_2$>

The dental porcelain as one embodiment of the present invention even has the composition containing $SiO_2$, $ZrO_2$, and an oxide of an alkaline earth metal satisfying the content of $CeO_2$ described above, but can suppress the thermal expansion coefficient from excessively increasing, and can suppress the occurrence of cracks in fusing on a ceramic base material, by the fact that the content of $SiO_2$ is 30.0% by mass or more based on 100% by mass of the dental porcelain. Furthermore, the content of $SiO_2$ that is 50.0% by mass or less based on 100% by mass of the dental porcelain enables the fusion thereof on a ceramic base material without firing at a firing temperature exceeding 1,000°C, and can regulate the firing temperature within the appropriate range, and thereby the coloration of the porcelain due to firing can be suppressed.

Accordingly, from the standpoint of further facilitating the effects of the present invention, the content of $SiO_2$ is preferably more than 30.0% by mass and 50.0% by mass or less, more preferably 33.0 to 48.0% by mass, and further preferably 37.0 to 45.0% by mass, based on 100% by mass of the dental porcelain.

<$ZrO_2$>

From the standpoint of further facilitating the effects of the present invention, the content of $ZrO_2$ is preferably 5.0 to 16.0% by mass, more preferably 6.0 to 15.0% by mass, and further preferably 7.5 to 14.5% by mass, based on 100% by mass of the dental porcelain.

<Oxide of Alkaline Earth Metal>

Examples of the oxide of an alkaline earth metal include one or more kind selected from the group consisting of MgO, CaO, SrO, and BaO.

From the standpoint of further facilitating the effects of the present invention, the dental porcelain preferably contains at least CaO or BaO, and more preferably contains at least CaO and BaO, as the oxide of an alkaline earth metal.

From the standpoint of further facilitating the effects of the present invention, it is further preferred that the dental porcelain contains at least CaO and BaO as the oxide of an alkaline earth metal, and has a content of CaO of 0.2 to 2.0% by mass based on 100% by mass of the dental porcelain, and a content of BaO of 1.0 to 8.0% by mass based on 100% by mass of the dental porcelain.

From the standpoint of further facilitating the effects of the present invention, the content of CaO is more preferably 0.3 to 1.5% by mass, and further preferably 0.4 to 1.0% by mass, based on 100% by mass of the dental porcelain.

From the standpoint of further facilitating the effects of the present invention, the content of BaO is more preferably 2.0 to 7.5% by mass, and further preferably 3.0 to 6.0% by mass, based on 100% by mass of the dental porcelain.

From the standpoint of further facilitating the effects of the present invention, the total content of the oxides of an alkaline earth metal in the dental porcelain is preferably 1.2 to 10.0% by mass, more preferably 2.3 to 9.0% by mass, and further preferably 3.4 to 7.0% by mass, based on 100% by mass of the dental porcelain.

From the standpoint of further facilitating the effects of the present invention, total content of $SiO_2$, $CeO_2$, $ZrO_2$, and the oxide of an alkaline earth metal in the dental porcelain is preferably 45.0 to 100% by mass, more preferably 50.0 to 99% by mass, further preferably 55.0 to 98.0% by mass, still further preferably 65.0 to 97.5% by mass, still more further preferably 70.0 to 95.0% by mass, even further preferably 75.0 to 90.0% by mass, and even still further preferably 78.0 to 86.0% by mass, based on 100% by mass of the dental porcelain.

<$Al_2O_3$, $K_2O$, and $Na_2O$>

From the standpoint of further facilitating the effects of the present invention, the dental porcelain preferably further contains at least one kind selected from $Al_2O_3$, $K_2O$, and $Na_2O$, and more preferably further contains $Al_2O_3$, $K_2O$, and $Na_2O$.

From the standpoint of further facilitating the effects of the present invention, it is further preferred that the dental porcelain further contains $Al_2O_3$, $K_2O$, and $Na_2O$, and has a content of $Al_2O_3$ of 2.0 to 8.0% by mass based on 100% by mass of the dental porcelain, a content of $K_2O$ of 1.0 to 7.0% by mass based on 100% by mass of the dental porcelain, and a content of $Na_2O$ of 4.0 to 10.0% by mass based on 100% by mass of the dental porcelain.

From the standpoint of further facilitating the effects of the present invention, the content of $Al_2O_3$ is more preferably 3.0 to 7.0% by mass, and further preferably 3.5 to 6.5% by mass, based on 100% by mass of the dental porcelain.

From the standpoint of further facilitating the effects of the present invention, the content of $K_2O$ is more preferably 2.0 to 6.5% by mass, and further preferably 3.0% by mass or more and less than 5.5% by mass, based on 100% by mass of the dental porcelain.

From the standpoint of further facilitating the effects of the present invention, the content of $Na_2O$ is more preferably 5.0 to 9.0% by mass, and further preferably 6.0 to 8.5% by mass, based on 100% by mass of the dental porcelain.

In the case where the dental porcelain contains at least one kind selected from $Al_2O_3$, $K_2O$, and $Na_2O$, from the standpoint of further facilitating the effects of the present invention, the total content of $SiO_2$, $CeO_2$, $ZrO_2$, the oxide of an alkaline earth metal, and the at least one kind selected from $Al_2O_3$, $K_2O$, and $Na_2O$ in the dental porcelain is preferably 50.0 to 100% by mass, more preferably 55.0 to 100% by mass, further preferably 65.0 to 98.0% by mass, still further preferably 70.0 to 100% by mass, still more further preferably 75.0 to 100% by mass, even further preferably 80.0 to 100% by mass, even still further preferably 83.5 to 100% by mass, even still more further preferably 90.0 to 100% by mass, yet further preferably 95.0 to 100% by mass, and yet still further preferably 98.0 to 100% by mass, based on 100% by mass of the dental porcelain.

In one embodiment of the present invention, it is preferred that $CeO_2$ contained in the dental porcelain is one that is blended as an opaquer. In other words, it is preferred that $CeO_2$ is $CeO_2$ that is derived from an opaquer.

In one embodiment of the present invention, in the case where $CeO_2$ contained in the dental porcelain is one that is blended as an opaquer as described above, the blending amount of $CeO_2$ blended as an opaquer is the same as in the description relating to the content of $CeO_2$ provided that the expression "content" is changed to "blended amount", and the expression "based on 100% by mass of the dental porcelain" is changed to "based on 100% by mass of raw material components of the dental porcelain", and the preferred amounts thereof are also the same.

In one embodiment of the present invention, it is preferred that $SiO_2$ contained in the dental porcelain is one that is blended as a glass component. In other words, it is preferred that $SiO_2$ is $SiO_2$ that is derived from a glass component.

In one embodiment of the present invention, in the case where $SiO_2$ contained in the dental porcelain is one that is blended as a glass component as described above, the blending amount of $SiO_2$ blended as a glass component is the same as in the description relating to the content of $SiO_2$ provided that the expression "content" is changed to "blended amount", and the expression "based on 100% by mass of the dental porcelain" is changed to "based on 100% by mass of raw material components of the dental porcelain", and the preferred amounts thereof are also the same.

In one embodiment of the present invention, it is preferred that $ZrO_2$ contained in the dental porcelain is one that is blended as a glass component. In other words, it is preferred that $ZrO_2$ is $ZrO_2$ that is derived from a glass component.

In one embodiment of the present invention, in the case where $ZrO_2$ contained in the dental porcelain is one that is blended as a glass component as described above, the preferred blending amount of $ZrO_2$ blended as a glass component is the same as in the description relating to the content of $ZrO_2$ provided that the expression "content" is changed to "blended amount", and the expression "based on 100% by mass of the dental porcelain" is changed to "based on 100% by mass of raw material components of the dental porcelain".

In one embodiment of the present invention, it is preferred that the oxide of an alkaline earth metal contained in the dental porcelain is one that is blended as a glass component. In other words, it is preferred that the oxide of an alkaline earth metal is an oxide of an alkaline earth metal that is derived from a glass component.

In one embodiment of the present invention, in the case where the oxide of an alkaline earth metal contained in the dental porcelain is one that is blended as a glass component as described above, the preferred total blending amount of the oxide of an alkaline earth metal blended as a glass component is the same as in the description relating to the content of the oxide of an alkaline earth metal provided that the expression "total content" is changed to "total blended amount", and the expression "based on 100% by mass of the dental porcelain" is changed to "based on 100% by mass of raw material components of the dental porcelain.

In one embodiment of the present invention, in the case where CaO described above as a preferred embodiment of the oxide of an alkaline earth metal is one that is blended as a glass component, the preferred blending amount of CaO blended as a glass component is the same as in the description relating to the content of CaO provided that the expression "content" is changed to "blended amount", and the expression "based on 100% by mass of the dental porcelain" is changed to "based on 100% by mass of raw material components of the dental porcelain".

Similarly, in one embodiment of the present invention, in the case where BaO described above as a preferred embodiment of the oxide of an alkaline earth metal is one that is blended as a glass component, the preferred blending amount of BaO blended as a glass component is the same as in the description relating to the content of BaO provided that the expression "content" is changed to "blended amount", and the expression "based on 100% by mass of the dental porcelain" is changed to "based on 100% by mass of raw material components of the dental porcelain".

In one embodiment of the present invention, in the case where $CeO_2$ contained in the dental porcelain is one that is blended as an opaquer, and $SiO_2$, $ZrO_2$, and the oxide of an alkaline earth metal contained therein are ones that are blended as glass components, the total blending amount of $CeO_2$ blended as an opaquer, and $SiO_2$, $ZrO_2$, and the oxide of an alkaline earth metal blended as glass components is the same as in the description relating to the total content of $SiO_2$, $CeO_2$, $ZrO_2$, and the oxide of an alkaline earth metal provided that the expression "total content" is changed to "total blended amount", and the expression "based on 100% by mass of the dental porcelain" is changed to "based on 100% by mass of raw material components of the dental porcelain".

In one embodiment of the present invention, in the case where the dental porcelain contains at least one kind selected from $Al_2O_3$, $K_2O$, and $Na_2O$, it is preferred that the one or more kinds of oxide selected from $Al_2O_3$, $K_2O$, and $Na_2O$ is one that is blended as a glass component. In other words, it is preferred that the one or more kinds of oxide selected from $Al_2O_3$, $K_2O$, and $Na_2O$ is one that is derived from a glass component.

In one embodiment of the present invention, in the case where $Al_2O_3$ that the dental porcelain may contain is one that is blended as a glass component as described above, the preferred blending amount of $Al_2O_3$ blended as a glass component is the same as in the description relating to the content of $Al_2O_3$ provided that the expression "content" is changed to "blended amount", and the expression "based on 100% by mass of the dental porcelain" is changed to "based on 100% by mass of raw material components of the dental porcelain".

Similarly, in one embodiment of the present invention, in the case where $K_2O$ that the dental porcelain may contain is one that is blended as a glass component as described above, the preferred blending amount of $K_2O$ blended as a glass component is the same as in the description relating to the content of $K_2O$ provided that the expression "content" is changed to "blended amount", and the expression "based on 100% by mass of the dental porcelain" is changed to "based on 100% by mass of raw material components of the dental porcelain".

Similarly, in one embodiment of the present invention, in the case where $Na_2O$ that the dental porcelain may contain is one that is blended as a glass component as described above, the preferred blending amount of $Na_2O$ blended as a glass component is the same as in the description relating to the content of $Na_2O$ provided that the expression "content" is changed to "blended amount", and the expression "based on 100% by mass of the dental porcelain" is changed to "based on 100% by mass of raw material components of the dental porcelain".

In one embodiment of the present invention, in the case where $CeO_2$ contained in the dental porcelain is one that is blended as an opaquer, $SiO_2$, $ZrO_2$, and the oxide of an alkaline earth metal contained therein are ones that are blended as glass components, and at least one kind selected from $Al_2O_3$, $K_2O$, and $Na_2O$ contained therein is one that is blended as a glass component, the total blending amount of $CeO_2$ blended as an opaquer, $SiO_2$, $ZrO_2$, and the oxide of an alkaline earth metal blended as glass components, and at least one kind selected from $Al_2O_3$, $K_2O$, and $Na_2O$ blended as a glass component is the same as in the description relating to the total content of $SiO_2$, $CeO_2$, $ZrO_2$, the oxide of an alkaline earth metal, and at least one kind selected from $Al_2O_3$, $K_2O$, and $Na_2O$ provided that the expression "total content" is changed to "total blended amount", and the expression "based on 100% by mass of the dental porcelain" is changed to "based on 100% by mass of raw material components of the dental porcelain".

In one embodiment of the present invention, the glass is preferably glass that contains $SiO_2$, $ZrO_2$, and the oxide of an alkaline earth metal, and more preferably glass that contains $SiO_2$, $ZrO_2$, the oxide of an alkaline earth metal, and at least one kind selected from $Al_2O_3$, $K_2O$, and $Na_2O$.

It is further preferred that the glass as the preferred embodiment is aluminosilicate glass containing the aforementioned components.

In the description herein, the "glass component" means one component constituting "glass" unless otherwise indicated.

The oxides shown as the "glass components" contained in the "glass" are the composition of the metal components contained in the "glass" shown in terms of metal oxides, and the "glass" may not necessarily contain the glass components in the form of the oxides. In the field of porcelains, such as glass and ceramics, as for the composition of the porcelain composition obtained by firing, it is common technical knowledge in this field to express the metal components contained in the composition in terms of metal oxides.

<Additional Components>

The dental porcelain may further contain a component derived from an additional glass component, a pigment, a fluorescent agent, an opaquer, and the like, as additional components other than the aforementioned components.

The additional component derived from a glass component is not particularly limited, as long as exhibiting the effects of the present invention, and examples thereof include at least one kind selected from the group consisting of $Li_2O$, $Sb_2O_3$, $B_2O_3$, $ZnO$, $SnO_2$, $Fe_2O_3$, $P_2O_5$, $Cl$, and $F$. In these components, the content of the oxide that can have multiple valences is calculated by converting into the valence of the component exemplified above. Specifically, for example, $FeO$ is calculated by converting into $1/2Fe_2O_3$.

Examples of the pigment and the fluorescent agent used include an oxide of at least one element selected from the group consisting of P, V, Cr, Mn, Fe, Co, Ni, Zn, Y, Sn, Sb, Bi, Pr, Sm, Eu, Gd, Tb, and Er.

Examples of the opaquer used include at least one crystalline compound selected from the group consisting of $ZrSiO_4$ and $SnO_2$. The pigment, the fluorescent agent, and the shielding material each may be one compound or may include multiple compounds.

In one embodiment of the present invention, the composition of the dental porcelain and the composition of glass capable of becoming raw materials of the dental porcelain can be confirmed in such a manner that the quantitative values of the contents of the oxides described above in the dental porcelain, which are measured with a combination of multiple analysis instruments capable of quantitatively determining the components by the known analysis methods, such as the fluorescent X-ray analysis, the atomic absorption analysis, and the inductively coupled plasma (ICP) emission spectroscopy, are summed and converted to 100% in total.

For example, the content ratios of $SiO_2$, $CeO_2$, $ZrO_2$, CaO, BaO, $Al_2O_3$, $K_2O$, and $Na_2O$ each can be measured, for example, by the fluorescent X-ray analysis. The content ratio of the $Li_2O$ component, which cannot be detected by the fluorescent X-ray analysis, can be measured, for example, by the atomic absorption analysis. The content ratio of the $B_2O_3$ component, which similarly cannot be detected by the fluorescent X-ray analysis, can be measured, for example, by the ICP emission spectroscopy.

In one embodiment of the present invention, for example, the composition calculated from the blending ratios of the components constituting the dental porcelain (in the case where the glass components are used, the composition of the glass containing the glass components, or the blending ratios of the raw materials) can be assumed to be the composition of the dental porcelain.

In the description herein, the oxides described above contained in the dental porcelain are the composition of the metal components contained in the dental porcelain in terms of metal oxides, and the dental porcelain may not necessarily contain the components in the form of the oxides. As described above, the same is applied to the glass capable of becoming raw materials of the dental porcelain, and in the field of porcelains, such as glass and ceramics, it is common technical knowledge in this field to express the metal components contained in the composition in terms of metal oxides, as for the composition of the porcelain composition obtained by firing.

<Method for producing Dental Porcelain>

One preferred embodiment of the method for producing the dental porcelain will be described. One example of the case where $CeO_2$ is mixed as an opaquer, and the other oxide components are mixed as glass components will be described.

(Production of Glass Raw Material)

The raw material glass of the porcelain is firstly produced. For example, raw materials (such as oxides and carbonates) corresponding to the oxide composition of the raw material glass are prepared and mixed. The mixed raw materials are then melted in a crucible preferably at 1,300 to 1,600°C for 2 to 8 hours, then the molten material is quenched in water to produce cullet, and the glass cullet is pulverized with a roll mill or a ball mill, and sieved to regulate into the prescribed particle diameter range.

(Production of Dental Porcelain)

Subsequently, the prepared primary glass powder (i.e., glass powder containing the glass components) and $CeO_2$ used as an opaquer are mixed. At this time, an inorganic pigment, a fluorescent agent, and the like may be added and mixed in some cases for the purpose of optimizing the firing temperature, the color tone and the fluorescence of the porcelain.

The condition in mixing is not particularly limited, and the components to be contained may be added all at once or in portions. The kneading device used for mixing may be an ordinary kneading device. Examples thereof include a mortar, a twin screw kneader (Twinmix), a triple screw kneader (Trimix), a kneader, and a planetary mixer.

The mixed powder is heat-treated preferably at 750 to 880°C for 0.5 to 2.5 hours. The heat treatment temperature is more preferably 770 to 850°C, further preferably 780 to 845°C, and still further preferably 790 to 840°C. The heat treatment temperature that is 880°C or less is preferred, for example, from the standpoint of preventing the problems, such as discoloration occurring in the dental porcelain, since $CeO_2$ is not melted in the glass more than necessary, and the effect of $CeO_2$ as the opaquer is easily exhibited. The heat treatment temperature that is 750°C or more is preferred, for example, from the standpoint of preventing the problems, such as an operation failure occurring in kneading and using the dental porcelain and a solvent, since $CeO_2$ can be easily fusion-bonded to the glass, preventing the fine powder of $CeO_2$ from being separated from the glass. The heat treatment time is more preferably 0.5 to 2 hours, and further preferably 1 to 2 hours.

The fired product having the glass and $CeO_2$ fusion-bonded to each other is then coarsely pulverized, and the coarsely pulverized product is further pulverized with a pot mill to the prescribed particle diameter range. Thereafter, the pulverized product is sieved to remove coarse particles therefrom, resulting in porcelain powder.

In one embodiment of the present invention, the average particle diameter of the porcelain powder is not particularly limited, as long as exhibiting the effects of the present invention, and is preferably 1 to 30 $\mu$m, more preferably 2 to 25 $\mu$m, and further preferably 3 to 20 $\mu$m.

The average particle diameter of the porcelain powder means the volume based average particle diameter (D50) that can be obtained through measurement by the laser diffraction-scattering method, and can be measured, for example, by the method described in the examples.

As shown in the preferred one embodiment of the method for producing the dental porcelain described above, examples of one embodiment of the present invention include a dental porcelain obtained by mixing glass containing glass components containing at least $SiO_2$, $ZrO_2$, and the oxide of an alkaline earth metal, and $CeO_2$ as an opaquer, and more preferably include a dental porcelain obtained by mixing glass containing glass components containing at least $SiO_2$, $ZrO_2$, and the oxide of an alkaline earth metal, and $CeO_2$ as an opaquer, and heat-treating the mixture. The blending amounts in mixing the components and the preferred amounts thereof each are independently the same as the blending amounts of the components described in the section of the dental porcelain described above.

$SiO_2$, $ZrO_2$, the oxide of an alkaline earth metal, and $CeO_2$ in the dental porcelain in this embodiment are the same as those described in the section of the dental porcelain of one embodiment of the present invention described above, and the preferred embodiments thereof are also the same. The contents of the components in the resulting dental porcelain and the preferred embodiments thereof are also the same. The mixing condition, the heat treatment condition after mixing, and the preferred embodiments thereof are also the same as described in the preferred one embodiment of the method for producing the dental porcelain described above.

<Thermal Expansion Coefficient of Dental Porcelain>

In one embodiment of the present invention, from the standpoint of further facilitating the suppression of the occurrence of cracks in the dental porcelain in fusing on a ceramic base material, a fired material obtained by firing the dental porcelain at a maximum firing temperature of 930°C preferably has a thermal expansion coefficient at 30 to 450°C of $7.8 \times 10^{-6}$ /°C or more and less than $10.0 \times 10^{-6}$ /°C, more preferably $7.8 \times 10^{-6}$ /°C or more and $9.8 \times 10^{-6}$ /°C or less, and further preferably $7.9 \times 10^{-6}$ /°C or more and $9.7 \times 10^{-6}$ /°C or less, measured according to ISO 6872 (2015) at a temperature rise rate of 10°C/min.

The value of the thermal expansion coefficient specifically means a value that is calculated by the method described in the examples.

<Transmittance of Dental Porcelain>

In one embodiment of the present invention, from the standpoint of further facilitating the improvement of the shielding capability of the dental porcelain, a measurement specimen having a thickness of 0.30 mm of a fired material obtained by firing the dental porcelain at a maximum firing temperature of 930°C preferably has a transmittance of light having a wavelength of 555 nm of 10.0% or less, more preferably 8.0% or less, further preferably 5.0% or less, still further preferably 3.0% or less, still more further preferably less than 1.0%, and even further preferably 0.9% or less, in a thickness direction. The lower limit value of the transmittance is not particularly limited, and may be 0%, or may be 0.1%, for example. As described above, the lower limit values and the upper limit values described in a stepwise manner each can be independently combined. For example, in one embodiment of the present invention, the transmittance of the dental porcelain is preferably 0 to 10.0%, more preferably 0 to 8.0%, further preferably 0 to 5.0%, still further preferably 0 to 3.0%, still more further preferably 0% or more and less than 1.0%, and even further preferably 0 to 0.9%, and for example, may be 0.1 to 10.0%, 0.1 to 8.0%, 0.1 to 5.0%, 0.1 to 3.0%, 0.1% or more and less than 1.0%, or 0.1 to 0.9%.

The value of the transmittance specifically means a value that is calculated by the method described in the examples.

<Brightness of Dental Porcelain>

In one embodiment of the present invention, from the standpoint of further facilitating the improvement of the aesthetics of the dental porcelain, a test piece having a thickness of 1.20 mm of a fired material obtained by firing the dental porcelain at a maximum firing temperature of 930°C preferably has a brightness L* of 90.0 or more, more preferably 91.0 or more, and further preferably 92.0 or more, obtained through colorimetry in the L*a*b* color space.

The upper limit value of the brightness L* is not particularly limited, and for example, may be 99.0, may be 98.0, or may be 97.0.

As described above, the lower limit values and the upper limit values described in a stepwise manner each can be independently combined. For example, in one embodiment of the present invention, the brightness L* of the dental porcelain may be 90.0 to 99.0, 90.0 to 98.0, 90.0 to 97.0, 91.0 to 99.0, 91.0 to 98.0, 91.0 to 97.0, 92.0 to 99.0, 92.0 to 98.0, or 92.0 to 97.0.

The value of the brightness L* specifically means a value that is calculated by the method described in the examples.

<Chromaticity of Dental Porcelain>

In one embodiment of the present invention, from the standpoint of further facilitating the improvement of the aesthetics of the dental porcelain, a test piece having a thickness of 1.20 mm of a fired material obtained by firing the dental porcelain at a maximum firing temperature of 930°C preferably has a chromaticity a* of -2.4 to +1.2, more preferably -2.3 to +1.1, and further preferably -2.2 to +1.0, obtained through colorimetry in the L*a*b* color space.

In one embodiment of the present invention, from the standpoint of further facilitating the improvement of the aesthetics of the dental porcelain, a test piece having a thickness of 1.20 mm of a fired material obtained by firing the dental porcelain at a maximum firing temperature of 930°C preferably has a chromaticity b* of -5.6 to +7.4, more preferably -5.5 to +7.3, and further preferably -5.4 to +7.2, obtained through colorimetry in the L*a*b* color space.

The values of chromaticities a* and b* mean values that are calculated by the method described in the examples.

[Method for producing Dental Porcelain Prosthesis]

Preferred embodiments also include a method for producing a dental prosthesis, including a step of coating the dental porcelain as one embodiment of the present invention on a zirconia base material, and a step of firing the dental porcelain at 880 to 980°C. The firing temperature of the dental porcelain is preferably 880 to 980°C, more preferably 900 to 960°C, and further preferably 920 to 940°C. The configuration is preferred since, for example, in the case where the porcelain is sintered on a ceramic base material, such as a zirconia crown, damages on the ceramic base material, such as a zirconia crown, can be suppressed. Furthermore, the configuration is preferred since the deformation of the ceramic base material due to overheating can be suppressed.

[Applications of Dental Porcelain]

The dental porcelain as one embodiment of the present invention can be favorably used as a dental porcelain applied to a ceramic base material, and can be used, for example, for producing a ceramic dental prosthesis, such as an inlay, an onlay, a laminate veneer, and a crown.

While a frame as a target on which the dental porcelain paste as one embodiment of the present invention is mounted is not particularly limited, preferred examples thereof include a ceramic frame, and preferred examples of the ceramic frame include a zirconia frame.

The application of the dental porcelain is not particularly limited, and preferred examples of the application thereof include a body porcelain (dentine color porcelain), a cervical porcelain, an incisal porcelain (enamel color porcelain), a translucent porcelain, an opaque porcelain, and a stain porcelain.

Examples of the other embodiment of the present invention also include use of the dental porcelain for treatment of teeth (for example, aesthetic dental treatment, missing tooth treatment, prosthetic restoration treatment, such as an artificial tooth, and carious treatment).

In all the embodiments described above, the kinds, the contents, and the like of the components can be appropriately changed, and the optional components can also be changed, e.g., added or deleted, based on the descriptions above. In all the embodiments described above, the values of the compositions and the characteristics of the dental porcelains can be appropriately changed and combined. Accordingly, one kind of the dental porcelain may be used alone, or two or more kinds thereof may be used in combination, as long as exhibiting the effects of the present invention.

The present invention encompasses embodiments which include various combinations of the configurations described above within the scope of the technical concept of the present invention, as long as exhibiting the effects of the present invention.

Examples

The present invention will be described more specifically with reference to examples below, but the present invention is not limited to the examples.

[Examples 1 to 7 and Comparative Examples 1 to 4]

Dental porcelains of Examples and Comparative Examples were produced in the following manner, and the characteristics thereof were evaluated.

<Production of Raw Material Glass>

Raw materials (e.g., silica, alumina, potassium carbonate, sodium carbonate, sodium nitrate, calcium carbonate, barium carbonate, barium fluoride, zirconium oxide, antimony trioxide, yttrium oxide, basic magnesium carbonate, lithium carbonate, and boric acid) were mixed to make the composition shown in Table 1 below in the production of primary glass powder. The mixed raw materials were melted in a crucible at 1,450°C for 4 hours. The molten material was quenched in water to produce glass cullet, and the glass cullet was pulverized with a ball mill, and sieved with a #100 stainless steel sieve to provide primary glass powder.

In Table 1, the additional components shown as "Others" include $Sb_2O_3$, $Y_2O_3$, $HfO_2$, $LiO_2$, $B_2O_3$, F, and the like.

[Table 1]

Table 1

| | Glass 1 | Glass 2 | Glass 3 | Glass 4 | Glass 5 | Glass 6 | Glass 7 | Glass 8 | Glass 9 |
|---|---|---|---|---|---|---|---|---|---|
| $SiO_2$ | 54.3 | 54.8 | 56.0 | 53.7 | 56.9 | 55.1 | 62.6 | 62.0 | 61.6 |
| $Al_2O_3$ | 6.8 | 7.1 | 6.4 | 6.7 | 7.0 | 18.8 | 10.4 | 5.3 | 15.3 |
| $K_2O$ | 5.8 | 6.1 | 5.5 | 5.7 | 6.0 | 11.0 | 7.8 | 6.0 | 12.8 |

(continued)

| | Glass 1 | Glass 2 | Glass 3 | Glass 4 | Glass 5 | Glass 6 | Glass 7 | Glass 8 | Glass 9 |
|---|---|---|---|---|---|---|---|---|---|
| $Na_2O$ | 9.5 | 10.3 | 9.2 | 9.6 | 10.1 | 12.0 | 14.2 | 6.6 | 7.5 |
| CaO | 0.9 | 1.0 | 0.9 | 0.9 | 1.0 | 0.5 | 0.5 | 0.1 | 0.8 |
| BaO | 5.9 | 6.4 | 5.8 | 6.0 | 6.3 | - | - | 1.4 | - |
| $ZrO_2$ | 16.5 | 12.0 | 16.1 | 14.6 | 12.4 | - | 1.2 | 13.7 | - |
| Others | 0.3 | 2.3 | 0.1 | 2.8 | 0.3 | 2.6 | 3.3 | 4.9 | 2.0 |
| Total | 100.0 | 100.0 | 100.0 | 100.0 | 100.0 | 100.0 | 100.0 | 100.0 | 100.0 |
| In the table, the contents of the components are in terms of % by mass. | | | | | | | | | |

<Production of Porcelain Powder>

The primary glass powder shown in Table 1 and $CeO_2$ as an opaquer were mixed at the ratio shown in Table 2. In Comparative Example 3, $TiO_2$ was also mixed as an opaquer. The mixed powder was heat-treated at 840°C for 2 hours.

Subsequently, the fired product having the glass and $CeO_2$ (in Comparative Example 3, the glass, $CeO_2$, and $TiO_2$) fusion-bonded to each other was coarsely pulverized with a jaw crusher, and the resulting coarsely pulverized material was further pulverized with a ball mill to an average particle diameter of 15 $\mu$m. Thereafter, the powder was sieved with a #100 stainless steel sieve to remove coarse particles, resulting in porcelain powder.

The compositions of the resulting porcelain powder are shown in Table 3.

The average particle diameter is the volume based average particle diameter (D50), which can be obtained by the laser diffraction-scattering method. In the laser diffraction-scattering method, specifically, the volume based average particle diameter (D50) can be measured with a laser diffraction particle size distribution analyzer (SALD-2300, available from Shimadzu Corporation) using a 0.2% sodium hexametaphosphate aqueous solution as the dispersion medium.

[Table 2]

Table 2

| | Example 1 | Example 2 | Example 3 | Example 4 | Example 5 | Example 6 | Example 7 |
|---|---|---|---|---|---|---|---|
| Glass 1 | 75.0 | 80.0 | 70.0 | - | - | - | - |
| Glass 2 | - | - | - | 75.0 | - | - | - |
| Glass 3 | - | - | - | - | 75.0 | - | - |
| Glass 4 | - | - | - | - | - | 82.0 | - |
| Glass 5 | - | - | - | - | - | - | 67.0 |
| Glass 6 | - | - | - | - | - | - | - |
| Glass 7 | - | - | - | - | - | - | - |
| Glass 8 | - | - | - | - | - | - | - |
| Glass 9 | - | - | - | - | - | - | - |
| $CeO_2$ | 25.0 | 20.0 | 30.0 | 25.0 | 25.0 | 18.0 | 33.0 |
| $TiO_2$ | - | - | - | - | - | - | - |
| Total | 100.0 | 100.0 | 100.0 | 100.0 | 100.0 | 100.0 | 100.0 |
| In the table, the amounts of the components (e.g., various glass, $CeO_2$, and $TiO_2$) are in terms of % by mass. (continued) | | | | | | | |

Table 2 (continued)

|  | Comparative Example 1 | Comparative Example 2 | Comparative Example 3 | Comparative Example 4 |
|---|---|---|---|---|
| Glass 1 | - | - | - | - |
| Glass 2 | - | - | - | - |
| Glass 3 | - | - | - | - |
| Glass 4 | - | - | - | - |
| Glass 5 | - | - | - | - |
| Glass 6 | 50.0 | - | - | - |
| Glass 7 | - | 92.2 | - | - |
| Glass 8 | - | - | 73.0 | - |
| Glass 9 | - | - | - | 75.0 |
| $CeO_2$ | 50.0 | 7.8 | 2.0 | 25.0 |
| $TiO_2$ | - | - | 25.0 | - |
| Total | 100.0 | 100.0 | 100.0 | 100.0 |
| In the table, the amounts of the components (e.g., various glass, $CeO_2$, and $TiO_2$) are in terms of % by mass. | | | | |

[Evaluation of Dental Porcelain]

The dental porcelains obtained in Examples and Comparative Examples were evaluated for the characteristics thereof in the following manner. The results obtained are shown in Table 3.

<Measurement of Transmittance of Dental Porcelain>

The porcelain powder was molded into a pellet and fired under condition of a maximum firing temperature of 930°C for 1 minute to provide a test piece, both surfaces of which were polished with #1000 waterproof abrasive paper ("C947H", available from Noritake Coated Abrasive Co., Ltd.), so as to provide a measurement specimen having a diameter of 11 mm and a thickness of 0.30 mm. In the measurement, the transmittance density was measured by applying light having a wavelength of 555 nm with a transmittance densitometer X-Rite (registered trademark) 361T(V) (available from X-Rite, Inc.), and the transmittance was calculated by substituting the measured value of the transmittance density into the following expression (1).

$$\text{Transmittance (\%)} = 10^{-\text{transmittance density}} \times 100 \qquad (1)$$

The firing for producing the test piece was performed specifically under condition of a drying time before firing of 5 minutes, an initial firing temperature of 600°C, a temperature rise rate of 45°C/min, a vacuum starting temperature of 600°C, and a vacuum degree of 96 kPa. At the time when the maximum firing temperature of 930°C was achieved, the atmosphere was released to the atmospheric pressure (vacuum release), and the test piece was retained for 1 minute, and after retaining, quenched to room temperature (25°C).

<Evaluation of Brightness and Chromaticity of Dental Porcelain>

The porcelain powder was molded into a pellet and fired under condition of a maximum firing temperature of 930°C for 1 minute to provide a test piece, both surfaces of which were polished with #1000 waterproof abrasive paper ("C947H", available from Noritake Coated Abrasive Co., Ltd.), so as to provide a test piece having a diameter of 14 mm and a thickness of 1.20 mm. The test piece produced was measured with a spectrophotometer CM-3610A (available from Konica Minolta, Inc.) with a D65 light source, the SCI measurement mode, a measured diameter/illumination diameter ratio of 8 mm/11 mm, and white background, from which the L* value as the brightness (color space) and the a* and b* values as chromaticities showing the hue and the chroma in the L*a*b* color coordinate system (JIS Z8781-4:2013, Colorimetry, Part 4, CIE 1976 L*a*b* color coordinate system) were read.
The firing for producing the test piece was performed specifically under the same condition as the condition shown in the section of the measurement of the transmittance of the dental porcelain.

<Evaluation of Presence or Absence of Coloration of Dental Porcelain>

A specimen was compared to a white porcelain "Cerabian (registered trademark) ZR Porcelain SB WHITE" as the reference sample of color tone, and the presence of coloration was visually confirmed. The specimen used for comparing color tone was the polished surface of the pellet used in the evaluation of the brightness and the chromaticity of the dental porcelain.

A specimen having no coloration observed was evaluated as "A: no coloration found", and a specimen having coloration observed was evaluated as "F: coloration found".

<Measurement of Thermal Expansion Coefficient of Dental Porcelain>

The thermal expansion coefficient was measured according to ISO 6872 (2015). The test piece used for the measurement was produced in such a manner that the porcelain powder was molded into a rod form (diameter: 5 mm) and fired under condition of a maximum firing temperature of 930°C for 1 minute, the upper and the lower surfaces of which were polished with #1000 waterproof abrasive paper ("C947H", available from Noritake Coated Abrasive Co., Ltd.) to make a length of 20 mm. The change in length of the test piece was measured within a range of 25 to 500°C at a temperature rise rate of 10°C/min with a thermomechanical analyzer TMA 8310 (available from Rigaku Corporation), and by using the data (thermal expansion curve) within a range of 30 to 450°C, the thermal expansion coefficient in a temperature range of 30 to 450°C was calculated.

The firing for producing the test piece was performed specifically under the same condition as the condition shown in the section of the measurement of the transmittance of the dental porcelain.

<Evaluation of Presence or Absence of Cracks>

The presence or absence of cracks in the porcelain was confirmed by using a fired pellet of $ZrO_2$ having 3% by mol of $Y_2O_3$ dissolved therein (shown as "3Y" in Table 3 below) and a fired pellet of $ZrO_2$ having 6% by mol of $Y_2O_3$ dissolved therein (shown as "6Y" in Table 3 below). The porcelain was coated on the two kinds of $ZrO_2$ pellets in a disk shape, and then fired under condition of 930°C for 1 minute, and then the state of fusion of the porcelain on the fired pellet was confirmed. The porcelain that was fused with the coated shape retained was evaluated as "A: no crack found", and the porcelain that had cracks formed therein or was partially or entirely peeled off from the fired pellet was evaluated as "F: cracks found".

The firing of the porcelain performed after coating the porcelain on the fired pellet was performed specifically under the same condition as the condition shown in the section of the measurement of the transmittance of the dental porcelain.

[Table 3]

Table 3

| | | Example 1 | Example 2 | Example 3 | Example 4 | Example 5 | Example 6 | Example 7 |
|---|---|---|---|---|---|---|---|---|
| Composition of porcelain | $SiO_2$ | 40.7 | 43.6 | 37.9 | 41.1 | 41.9 | 44.0 | 38.1 |
| | $Al_2O_3$ | 5.1 | 5.4 | 4.8 | 5.3 | 4.8 | 5.5 | 4.7 |
| | $K_2O$ | 4.4 | 4.6 | 4.1 | 4.6 | 4.1 | 4.7 | 4.0 |
| | $Na_2O$ | 7.1 | 7.6 | 6.7 | 7.7 | 6.9 | 7.9 | 6.8 |
| | CaO | 0.7 | 0.7 | 0.6 | 0.8 | 0.7 | 0.7 | 0.7 |
| | BaO | 4.4 | 4.7 | 4.1 | 4.8 | 4.4 | 4.9 | 4.2 |
| | $ZrO_2$ | 12.4 | 13.2 | 11.6 | 9.0 | 12.1 | 12.0 | 8.3 |
| | $CeO_2$ | 25.0 | 20.0 | 30.0 | 25.0 | 25.0 | 18.0 | 33.0 |
| | $TiO_2$ | - | - | - | - | - | - | - |
| | Others | 0.2 | 0.2 | 0.2 | 1.7 | 0.1 | 2.3 | 0.2 |
| | Total | 100.0 | 100.0 | 100.0 | 100.0 | 100.0 | 100.0 | 100.0 |
| Characteristics | Transmittance (%) (Shielding capability) | 0.9 | 4.1 | 0.6 | 2.1 | 0.7 | 8.0 | 0.2 |
| | Presence of coloration | A | A | A | A | A | A | A |
| | $L^*$ | 92.9 | 92.6 | 93.2 | 93.2 | 93.2 | 93.1 | 94.1 |
| | $a^*$ | -1.0 | -1.2 | -1.1 | -1.0 | -1.1 | -0.9 | -0.3 |
| | $b^*$ | -1.1 | -1.5 | -1.0 | -0.5 | -0.9 | -0.4 | 1.9 |
| | Thermal expansion coefficient ($\times 10^{-6}$/°C) | 8.8 | 8.5 | 9.4 | 9.6 | 8.2 | 8.0 | 9.8 |
| | Presence of cracks on 3Y | A | A | A | A | A | A | A |
| | Presence of cracks on 6Y | A | A | A | A | A | A | F |

In the table, the contents of the components in the "composition of porcelain" are in terms of % by mass.

(continued)

Table 3 (continued)

| | | Comparative Example 1 | Comparative Example 2 | Comparative Example 3 | Comparative Example 4 |
|---|---|---|---|---|---|
| Composition of porcelain | $SiO_2$ | 27.5 | 57.7 | 45.2 | 46.2 |
| | $Al_2O_3$ | 9.4 | 9.6 | 3.9 | 11.5 |
| | $K_2O$ | 5.5 | 7.2 | 4.4 | 9.6 |
| | $Na_2O$ | 6.0 | 13.1 | 4.8 | 5.6 |
| | CaO | 0.3 | 0.5 | 0.1 | 0.6 |
| | BaO | - | - | 1.0 | - |
| | $ZrO_2$ | - | 1.1 | 10.0 | - |
| | $CeO_2$ | 50.0 | 7.8 | 2.0 | 25.0 |
| | $TiO_2$ | - | - | 25.0 | - |
| | Others | 1.3 | 3.0 | 3.6 | 1.5 |
| | Total | 100.0 | 100.0 | 100.0 | 100.0 |
| Characteristics | Transmittance (%) (Shielding capability) | 1.0 | 12.6 | 0.1 | 1.0 |
| | Presence of coloration | A | A | F (blackened) | F (yellowed) |
| | $L^*$ | 95.1 | 95.2 | 82.1 | 90.4 |
| | $a^*$ | 0.2 | 0.2 | 1.6 | 1.3 |
| | $b^*$ | 3.4 | 3.5 | 9.5 | 14.1 |
| | Thermal expansion coefficient ($\times 10^{-6}$/°C) | 12.0 | 9.5 | 8.9 | 9.8 |
| | Presence of cracks on 3Y | F | A | A | A |
| | Presence of cracks on 6Y | F | A | A | A |

In the table, the contents of the components in the "composition of porcelain" are in terms of % by mass.

It is understood from the results in Table 3 that the dental porcelains of Examples 1 to 7 have an excellent shielding capability, can suppress the discoloration of the porcelain in firing, and can suppress the occurrence of cracks in fusing on a ceramic base material.

On the other hand, it is understood therefrom that the dental porcelain of Comparative Example 1 having a content of $SiO_2$ of less than 30.0% by mass and containing no $ZrO_2$ has a large thermal expansion coefficient, and cracks are formed therein.

It is understood therefrom that the dental porcelain of Comparative Example 2 having a content of $SiO_2$ exceeding 50.0% by mass, and a content of $CeO_2$ of less than 10.0% by mass has a large transmittance, and is inferior in shielding capability.

It is understood therefrom that the dental porcelain of Comparative Example 3 having a content of $CeO_2$ of less than 10.0% by mass, and having $TiO_2$ mixed therein as an opaquer causes discoloration (blackened) in firing.

It is understood therefrom that the dental porcelain of Comparative Example 4 containing no $ZrO_2$ causes discoloration (yellowed) in firing.

Industrial Applicability

The dental porcelain of the present invention can be applied to a dental prosthesis and the like.

In recent years, there is an increasing demand of ceramic dental crowns and the like, and also an increasing individual aesthetic demand, from which the use of dental porcelains is expected to increase. Under the circumstances, the dental porcelain of the present invention is useful as a dental porcelain that is applied to a base material using ceramics, such as zirconia.

**Claims**

1. A dental porcelain comprising $SiO_2$, $CeO_2$, $ZrO_2$, and an oxide of an alkaline earth metal, having

   a content of the $SiO_2$ of 30.0 to 50.0% by mass based on 100% by mass of the dental porcelain, and
   a content of the $CeO_2$ of 10.0 to 50.0% by mass based on 100% by mass of the dental porcelain.

2. The dental porcelain according to claim 1, wherein the dental porcelain has a content of the $ZrO_2$ of 5.0 to 16.0% by mass based on 100% by mass of the dental porcelain.

3. The dental porcelain according to claim 1 or 2, which further comprises $Al_2O_3$, $K_2O$, and $Na_2O$, and has

   a content of the $Al_2O_3$ of 2.0 to 8.0% by mass based on 100% by mass of the dental porcelain,
   a content of the $K_2O$ of 1.0 to 7.0% by mass based on 100% by mass of the dental porcelain, and
   a content of the $Na_2O$ of 4.0 to 10.0% by mass based on 100% by mass of the dental porcelain.

4. The dental porcelain according to any one of claims 1 to 3, wherein the dental porcelain comprises at least CaO and BaO as the oxide of an alkaline earth metal, and has

   a content of the CaO of 0.2 to 2.0% by mass based on 100% by mass of the dental porcelain, and
   a content of the BaO of 1.0 to 8.0% by mass based on 100% by mass of the dental porcelain.

5. The dental porcelain according to any one of claims 1 to 4, wherein the dental porcelain has a total content of the $SiO_2$, the $CeO_2$, the $ZrO_2$, and the oxide of an alkaline earth metal of 70.0 to 95.0% by mass based on 100% by mass of the dental porcelain.

6. The dental porcelain according to any one of claims 1 to 5, wherein the $CeO_2$ is $CeO_2$ that is derived from an opaquer.

7. The dental porcelain according to any one of claims 1 to 6, wherein a fired material obtained by firing the dental porcelain at a maximum firing temperature of 930°C has a thermal expansion coefficient at 30 to 450°C of $7.8 \times 10^{-6}$ /°C or more and less than $10.0 \times 10^{-6}$ /°C measured according to ISO 6872 (2015) at a temperature rise rate of 10°C/min.

8. The dental porcelain according to any one of claims 1 to 7, wherein a measurement specimen having a thickness of 0.30 mm of a fired material obtained by firing the dental porcelain at a maximum firing temperature of 930°C has a transmittance of light having a wavelength of 555 nm of 10.0% or less in a thickness direction.

9. The dental porcelain according to any one of claims 1 to 8, wherein a test piece having a thickness of 1.20 mm of a fired material obtained by firing the dental porcelain at a maximum firing temperature of 930°C has a brightness L* of 90.0 or more obtained through colorimetry in the L*a*b* color space.

10. A method for producing a dental prosthesis, comprising a step of coating the dental porcelain according to any one of claims 1 to 9 on a zirconia base material, and a step of firing the dental porcelain at 880 to 980°C.

## INTERNATIONAL SEARCH REPORT

| International application No. |
| --- |
| **PCT/JP2023/046285** |

**A. CLASSIFICATION OF SUBJECT MATTER**

*A61K 6/833*(2020.01)i; *A61C 13/003*(2006.01)i; *A61K 6/818*(2020.01)i; *A61K 6/822*(2020.01)i; *A61L 27/10*(2006.01)i; *C03C 3/062*(2006.01)i; *C03C 3/078*(2006.01)i; *C03C 3/085*(2006.01)i; *C03C 3/087*(2006.01)i; *C04B 35/18*(2006.01)i
FI: A61K6/833; A61K6/818; A61K6/822; A61L27/10; A61C13/003; C03C3/062; C03C3/078; C03C3/085; C03C3/087; C04B35/18

According to International Patent Classification (IPC) or to both national classification and IPC

**B. FIELDS SEARCHED**

Minimum documentation searched (classification system followed by classification symbols)

A61K6/833; A61C13/003; A61K6/818; A61K6/822; A61L27/10; C03C3/062; C03C3/078; C03C3/085; C03C3/087; C04B35/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2016-505541 A (DEGUDENT GMBH) 25 February 2016 (2016-02-25)<br>entire text, all drawings | 1-10 |
| A | JP 2006-89502 A (TEC VENTURES INC.) 06 April 2006 (2006-04-06)<br>entire text, all drawings | 1-10 |
| A | JP 2005-187436 A (NORITAKE CO., LIMITED) 14 July 2005 (2005-07-14)<br>entire text, all drawings | 1-10 |
| A | JP 2003-503431 A (JENERIC/PENTRON INCORPORATED) 28 January 2003 (2003-01-28)<br>entire text, all drawings | 1-10 |
| A | JP 2010-519986 A (IVOCLAR VIVADENT AG) 10 June 2010 (2010-06-10)<br>entire text, all drawings | 1-10 |

☑ Further documents are listed in the continuation of Box C. ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **05 March 2024** | **19 March 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
|---|
| **PCT/JP2023/046285** |

| C. | DOCUMENTS CONSIDERED TO BE RELEVANT | |
|---|---|---|
| Category* | Citation of document, with indication, where appropriate, of the relevant passages | Relevant to claim No. |
| A | JP 10-94550 A (NORITAKE CO., LIMITED) 14 April 1998 (1998-04-14)<br>entire text, all drawings | 1-10 |
| A | JP 2007-126360 A (KABUSHIKI KAISHA SHOFU) 24 May 2007 (2007-05-24)<br>entire text, all drawings | 1-10 |
| A | JP 2009-185001 A (TOKUYAMA DENTAL CORP.) 20 August 2009 (2009-08-20)<br>entire text, all drawings | 1-10 |
| A | US 2019/0038387 A1 (CHU, Christopher) 07 February 2019 (2019-02-07)<br>entire text, all drawings | 1-10 |

Form PCT/ISA/210 (second sheet) (July 2022)

## INTERNATIONAL SEARCH REPORT
### Information on patent family members

International application No.

**PCT/JP2023/046285**

| Patent document cited in search report | | | Publication date (day/month/year) | Patent family member(s) | | | Publication date (day/month/year) |
|---|---|---|---|---|---|---|---|
| JP | 2016-505541 | A | 25 February 2016 | WO | 2014/082969 | A1 | |
| | | | | entire text, all drawings | | | |
| | | | | DE | 102012111683 | A1 | |
| | | | | CA | 2892284 | A1 | |
| | | | | CN | 104955436 | A | |
| JP | 2006-89502 | A | 06 April 2006 | US | 5507981 | A | |
| | | | | entire text, all drawings | | | |
| | | | | WO | 1995/032678 | A2 | |
| | | | | EP | 1005841 | A1 | |
| | | | | CA | 2359063 | A1 | |
| | | | | CN | 1130860 | A | |
| JP | 2005-187436 | A | 14 July 2005 | US | 2006/0261503 | A1 | |
| | | | | entire text, all drawings | | | |
| | | | | WO | 2005/007009 | A1 | |
| | | | | EP | 1654998 | A1 | |
| | | | | EP | 2377488 | A1 | |
| | | | | CN | 1822796 | A | |
| | | | | KR | 10-2006-0056948 | A | |
| JP | 2003-503431 | A | 28 January 2003 | US | 6428614 | B1 | |
| | | | | entire text, all drawings | | | |
| | | | | US | 2003/0122270 | A1 | |
| | | | | WO | 2001/001925 | A1 | |
| JP | 2010-519986 | A | 10 June 2010 | US | 2011/0030423 | A1 | |
| | | | | entire text, all drawings | | | |
| | | | | US | 8987153 | B2 | |
| | | | | US | 2015/0183681 | A1 | |
| | | | | WO | 2008/106958 | A2 | |
| | | | | DE | 102007011337 | A1 | |
| | | | | CN | 101932298 | A | |
| | | | | CN | 103395982 | A | |
| JP | 10-94550 | A | 14 April 1998 | (Family: none) | | | |
| JP | 2007-126360 | A | 24 May 2007 | (Family: none) | | | |
| JP | 2009-185001 | A | 20 August 2009 | (Family: none) | | | |
| US | 2019/0038387 | A1 | 07 February 2019 | (Family: none) | | | |

Form PCT/ISA/210 (patent family annex) (July 2022)

**Patent documents cited in the description**

- JP 10094550 A

- JP 2007126360 A